# EUROPÄISCHE PATENTSCHRIFT

(11) **EP 1 217 377 B1**
(45) Veröffentlichungstag und Bekanntmachung des Hinweises auf die Patenterteilung: **14.10.2009**
(21) Anmeldenummer: 01128431.2
(22) Anmeldetag: 05.12.2001
(51) Int. Cl.: G01N 33/574, C12Q 1/68

(54) **Verfahren zur spezifischen Detektion von Tumorzellen und ihren Vorstufen in Gebärmutterhalsabstrichen durch simultane Messung von mindestens 2 verschiedenen molekularen Markern**
Process for the specific detection of tumour cells and their precursors in cervical smears through simultaneous measurement of at least two different molecular markers
Procédé pour la détection de cellules tumorales et leurs précurseurs dans des frottis du col de l'utérus par la mesure simultanée d'au moins deux marqueurs moléculaires différents

(30) Priorität: 18.12.2000 DE 10063179
(43) Veröffentlichungstag der Anmeldung: 26.06.2002
(73) Patentinhaber: Siemens Healthcare Diagnostics GmbH, 65760 Eschborn (DE)
(72) Erfinder: Hennig, Guido, Dr., 50859 Köln (DE); Wirtz, Ralph, Dr., 50674 Köln (DE); Doth, Margit, Dr., 51371 Leverkusen (DE); Bohmann, Kerstin, Dr., 47798 Krefeld (DE); Unger, Silvia, Dr., 69120 Heidelberg (DE)
(74) Vertreter: Maier, Daniel Oliver

(56) Entgegenhaltungen:
- WO-A-00/24760
- WO-A-97/21979
- WO-A-99/21014
- US-A- 5 544 650
- US-A- 5 932 412
- US-A- 6 005 256
- GOMEZ F ET AL: "Simultaneous detection of antigens and specific DNA sequences of human papillomavirus in uterine cervical biopsy specimens. Description of a double-labelling technique" EUROPEAN JOURNAL OF HISTOCHEMISTRY, Bd. 41, Nr. 4, 1997, Seiten 255-259, XP001069548 ISSN: 1121-760X
- WILLIAMS GARETH H ET AL: "Improved cervical smear assessment using antibodies against proteins that regulate DNA replication" PROCEEDINGS OF THE NATIONAL ACADEMY OF SCIENCES OF THE UNITED STATES, Bd. 95, Nr. 25, Dezember 1998 (1998-12), Seiten 14932-14937, XP002261029 Dec., 1998 ISSN: 0027-8424
- MUNIRAJAN A K ET AL: "THE STATUS OF HUMAN PAPILLOMAVIRUS AND TUMOR SUPPRESSOR GENES P53 AND P16 IN CARCINOMAS OF UTERINE CERVIX FROM INDIA" GYNECOLOGIC ONCOLOGY, ACADEMIC PRESS, LONDON, GB, Bd. 69, Nr. 3, Juni 1998 (1998-06), Seiten 205-209, XP009015130 ISSN: 0090-8258
- KERSEMAEKERS A M ET AL: "Oncogene alterations in carcinomas of the uterine cervix: overexpression of the epidermal growth factor receptor is associated with poor prognosis." CLINICAL CANCER RESEARCH: AN OFFICIAL JOURNAL OF THE AMERICAN ASSOCIATION FOR CANCER RESEARCH. UNITED STATES MAR 1999, Bd. 5, Nr. 3, März 1999 (1999-03), Seiten 577-586, XP002261030 ISSN: 1078-0432
- PILLAI M RADHAKRISHNA ET AL: "The presence of human papillomavirus-16/-18 E6, p53, and Bcl-2 protein in cervicovaginal smears from patients with invasive cervical cancer" CANCER EPIDEMIOLOGY BIOMARKERS AND PREVENTION, Bd. 5, Nr. 5, 1996, Seiten 329-335, XP009020437 ISSN: 1055-9965
- MITRA A B ET AL: "ERBB2 (HER2/neu) Oncogene Is Frequently Amplified in Squamous Cell Carcinoma of the Uterine Cervix" CANCER RESEARCH, Bd. 54, Nr. 3, 1994, Seiten 637-639, XP001156107 ISSN: 0008-5472
- SKOMEDAL H ET AL: "ABERRANT EXPRESSION OF THE CELL CYCLE ASSOCIATED PROTEINS TP53, MDM2, P21, P27, CDK4, CYCLIN D1, RB AND EGFR IN CERVICAL CARCINOMAS" GYNECOLOGIC ONCOLOGY, ACADEMIC PRESS, LONDON, GB, Bd. 73, Nr. 2, Mai 1999 (1999-05), Seiten 223-228, XP001086409 ISSN: 0090-8258
- BUSMANIS I: "BIOMARKERS IN CARCINOMA OF THE CERIVIX: EMPHASIS ON TISSUE-RELATED FACTORS AND THEIR POTENTIAL PORGNOSTIC FACTORS" ANNALS OF THE ACADEMY OF MEDICINE, SINGAPORE, ACADEMY OF MEDICINE, SG, Bd. 27, Nr. 5, September 1998 (1998-09), Seiten 671-675, XP001095027 ISSN: 0304-4602
- BERGERON C ET AL: "HUMAN PAPILLOMAVIRUSES ASSOCIATED WITH CERVICAL INTRAEPITHELIAL NEOPLASIA GREAT DIVERSITY AND DISTINCT DISTRIBUTION IN LOW AND HIGH-GRADE LESIONS" AMERICAN JOURNAL OF SURGICAL PATHOLOGY, Bd. 16, Nr. 7, 1992, Seiten 641-649, XP009020964 ISSN: 0147-5185
- ROTHMANN ET AL.: "Potential use of spectral image analysis for the quantitative evaluation of estrogen receptors in breast cancer" HISTOLOGY AND HISTOPATHOLOGY, Bd. 15, 2000, Seiten 1051-1057,

## Beschreibung

### Stand der Technik

Nach wie vor zählen Krebserkrankungen zu den häufigsten Todesursachen weltweit. Es besteht ein klinischer Bedarf an der Früherkennung und spezifischen Detektion von Krebs und Krebsvorstufen, um die Entwicklung zum Tumor durch frühzeitige therapeutische Intervention zu verhindern. Aus diesem Grunde werden bereits seit den fünfziger Jahren Vorsorgeprogramme für verschiedene Karzinome (besonders Cervix, Brust und Darm) angeboten, welche in zahlreichen Ländern zu sinkenden Mortalitätsraten geführt haben.

Die Vorsorgeuntersuchung beim Gebärmutterhalskrebs der Frau basiert im Wesentlichen auf der morphologisch-zytologischen Untersuchung von Zellabstrichen des Gebärmutterhalses, dem sogenannten PAP Test. Dieser Test weist allerdings eine limitierte Sensitivität (bis zu 40% falsch negative Diagnosen, Duggan et al., 1998, Eur J Gynaecol Oncol; 19:209-214; Bishop et al., 1996, Buss Pan Am Health Organ, 30,378-86) auf. Des weiteren werden bis zu 10% der Abstriche als ASCUS klassifiziert (atypical squamous cells of undetermined significance), d.h. eine klare Einordnung in normal, niedrig- oder hochgradige Läsion bzw. Tumor ist nicht möglich. Erfahrungsgemäß befinden sich in dieser ASCUS Population aber bis zu 10% wahre Läsionen, die somit auch übersehen werden (Manos et al., 1999, JAMA 281, 1605-1610). Bei der Entdeckung abnormaler Zellen werden zusätzliche diagnostische Verfahren wie Kolposkopie oder eine Biopsieentnahme durchgeführt, anhand derer eine verbesserte Diagnose möglich ist und eine Therapieentscheidung, wie z.B. chirurgische Entnahme der pathologischen Region oder Lasertherapie, getroffen werden kann.

In den letzten Jahren wurden zahlreiche Gene identifiziert, die aufgrund von Mutationen zu einer verändertem Expression ihrer Proteine führen und somiteine Rolle bei der Krebsentstehung spielen. Potentiell können die korrespondierenden Proteine als diagnostische Marker sowohl im Serum als auch auf zellulärer Ebene nachgewiesen werden.. Die Proteine sind an physiologischen Regulationsprozessen in der Zelle, wie z.B. der Wachstumskontrolle (Proliferationsgene, z.B. Ki67, mcm-5; Onkogene und Tumorsuppressorgene, z.B. p16, EGF-Rezeptor, her2/neu, mdm-2), Apoptose (natürlicher Zelltod, z.B. p53, bcl-2), Reparatur von DNA (msh-1) und Zelladhäsion (E-Cadherin, ß-catenin, APC) beteiligt. Zum Nachweis der Marker eignen sich z.B. immunhisto- und immuncytochemische Detektionsmethoden mit spezifischen Antikörpern gegen diese Proteine (van Noorden, 1986, Immuncytochemistry, Modern Methods and Applications , 2^{nd} edition, Wright, Bristol, 26-53).

Bei bestimmten Krebsarten ist darüber hinaus das Auftreten von Viren signifikant mit der Krebsentstehung assoziiert. Virale DNA wird durch Hybridisierungsverfahren nachgewiesen und dient ebenfalls als diagnostischer Marker, z.B. beim Nachweis humaner Papillomviren in Gebärmutterhalsabstrichen (Detektonskits erhältlich von mehreren Anbietern, z.B. Digene, DAKO, BioGenex, ENZO).

Es ist vielfach gezeigt, dass ein einzelner Marker keine ausreichende Spezifität hinsichtlich der Erkennung von krankhaft veränderten Zellen erreicht, da er zum Teil auch in gesunden Zellen vorhanden ist. Dies basiert auf der Tatsache, dass Marker Proteine sind, die an physiologischen Regulationsprozessen auch in gesunden Zellen beteiligt sind. So ist z.B. HPV in nahezu allen Tumoren des Cervix, jedoch auch in vielen normalen Cervixepithelien nachzuweisen, d.h. ein Test zum Nachweis von HPV weist eine hohe Sensitivität jedoch nur eine geringe Spezifität auf (z.B. Cuzick, 2000, JAMA, 283, 108-109). Williams et al. (1998, PNAS, 95, 14932-14937) beschreiben den DNA Replikationsmarker mcm-5, welcher 28 getestete Läsionen/Tumore des Cervix (n=58) detektiert, aber 2 von 28 Normalabstrichen falsch positiv anfärbt. Sano et al. (Pathology Intl., 1998, 48, 580-585) beschreiben, dass der Marker p16 unspezifisch 3 von 15 normalen Cervixbiopsien anfärbt.

In der Cytologie/Pathologie ist es zunehmend von Interesse mehrere Marker in einer biologischen Probe nachzuweisen. Zum einen steht damit mehr Information zur Verfügung, zum anderen können unspezifische Färbungen einzelner Marker als solche eher erkannt werden. Traditionell werden zu diesem Zweck serielle Schnitte einer vorhandenen Biopsie bzw. mehrfache Präparationen einer Körperprobe (z.B. Gebärmutterhalsabstrich in Fixierflüssigkeit) hergestellt und mit jeweils einem bestimmten Marker gefärbt. Dieses Verfahren hat mehrere Nachteile: hoher Materialverbrauch der meist limitierten Patientenprobe und von Färbereagenzien, hoher Zeitaufwand und Kosten sowie keine Möglichkeit der eindeutigen Co-Lokalisierung von zwei oder mehreren Markern in einer Zelle. Der simultane Nachweis von zwei oder mehreren verschiedenen biologischen Markern auf einer biologischen Probe ist bereits in der Literatur beschrieben (DAKO, Practical Guide for Immunoenzymatic Double Staining Methods; Gomez et al, Eur. J. Histochem., 1997, 41, 255-259), wird jedoch in der Routinediagnostik bisher nicht angewendet. Terhavauta et al. (Cytopathology, 1994, 5, 282-293) verwenden eine Doppelfärbung von p53 und Ki67 auf Biopsien von Cervix-Läsionen und können beide Marker in Basal- und Parabasalzellen von HPV positiven und negativen Läsionen z.T. in einer Zelle nachweisen. Die beschriebene Doppelfärbung wurde auf Cervix-Biopsien durchgeführt und ist nicht übertragbar auf Gebärmutterhalsabstriche. Ebenso ist die Zellzusammensetzung eines Abstrichs, der kaum Basal- und Parabasalzellen aufweist, unterschiedlich zu einer Biopsie. Nach unseren Erkenntnissen ist mit der beschriebenen Markerkombination auf Cervixabstrichen kein diagnostisch auswertbares Ergebnis zu erzielen. Darüber hinaus benutzen die Autoren die gewonnene Information der Doppelfärbung nicht, um durch Kombination der Ergebnisse eine verbesserte Aussage über das Auftreten von Tumorzellen in dieser Probe treffen.

Rao et al. (1998, Cancer Epidemology, Biomarkers and Evolution, 7, 1027-1033) beschreiben eine Mehrfachfärbung von verschiedenen tumorassoziierten Markern (DNA-Gehalt, G-Aktin und p53) auf Nadelpunktionsmaterial (FNA, fine needle aspirates) der Brust. Sie interpretieren ihre Ergebnisse dahingehend, dass durch die Verwendung mehrerer Marker eine höhere klinische Spezifität erreicht wird, als die Betrachtung der einzelnen Marker und somit bei der Früherkennung von Brustkrebs eine wichtige Rolle spielen können. Die von den Autoren angeführte Mehrfachfärbung ist auf Nadelpunktionsmaterial der Brust beschränkt. Es fehlt der Ansatz zur Nutzung der Färbeinformation im Hinblick auf eine automatisierte Detektion von Tumorbereichen. Darüber hinaus ist die beschriebene Färbemethodik nicht auf andere biologische Materialien (i.e. Biopsien, Gebärmutterhalsabstriche) auf Grund der unterschiedlichen Stoffeigenschaften zu übertragen.

### Automatisierung:

Eine weitere Verbesserung der momentanen klinischen Tumordiagnostik besteht sowohl in der Automatisierung der Probenpräparation und -färbung, als auch in der automatisierten Bildanalyse mit Diagnosestellung. Dies führt neben einer zeitlichen und personellen Ersparnis auch zu einer objektiveren und damit einheitlicheren Diagnose. Die Anfärbung von biologischen Markern in Tumorzellen oder deren Vorläufern durch Fluoreszenz oder chromogene Farbstoffe ermöglicht eine Quantifizierung und somit automatisierte Auslesung der Signale. Es gibt bereits Systeme, die eine automatisierte Durchführung von Färbeprotokollen erlauben, allerdings ohne automatisierte Diagnose durch Bildanalyse (LeNeel et al, 1998, Clin Chim Acta, 278, 185-192). Am weitesten fortgeschritten ist die automatisierte Detektion von Tumorzellen und deren Vorläufern mit Hilfe morphologischer Bildinformationen bei Cervix-Abstrichen (Sawaya et al, 1999,Clinical Obstetrics and Gynecology, 42, 922-938; Stoler, 2000, Mod. Pathol., 13, 275-284).

Boon et al. (1995, Diagn. Cytopathol., 13, 423-428) nutzen neben der automatisierten morphologischen Detektion von abnormalen Zellen in Gebärmutterhalsabstrichen mit dem PAPNET System zusätzlich die immunhistochemische Anfärbung von proliferierenden Zellen mit Ki67-Antikörpern. Der Gebrauch dieses einen molekularen Markers ermöglicht jedoch keine eindeutige Unterscheidung zwischen gutartigen proliferierenden und kanzerogenen Zellen.

In dem Patent von Boon *et al.* (US-5544650) ist beschrieben, dass eine Färbung mit einem immunhistochemischen Marker die automatische Detektion von proliferierenden (kanzerogene und nicht-kanzerogene) Zellen in einer Probe erleichtert und in einem semi-automatischen Prozess der kontrollierende Pathologe/Cytologe entscheidet, ob es sich bei den gefärbten Zellen wirklich um kanzerogene Zellen handelt.

Das Patent US-6005256 von McGlynn und Akkapeddi beschreibt ausführlich ein Gerät und ein Verfahren zur simultanen Detektion von mehreren fluoreszenzmarkierten Markern in einer Körperprobe, auch zur Identifikation von Krebszellen, ohne aber auf eine konkrete Anwendung in der Krebsdiagnostik einzugehen bzw. entsprechende Markerkombinationen mit erhöhter Spezifität zu benennen.

Von Ampersand Medical Systems Group (www.ampersandmedical.com) ist bekannt, dass sie ein neues Screening System für Cervix-Abstriche (InPath) entwickeln, welches neben einer eigenen Probenaufbereitung auch die Fluoreszenzdetektion von nicht genannten biologischen Markern beinhaltet.

### Aufgabe

Der vorliegenden Erfindung beinhaltet Verfahren mit denen Karzinomzellen oder deren Vorstufen in präparierten Gebärmutterhalsabstrichen frühzeitig und zuverlässiger als bisher diagnostiziert werden können.

Erfindungsgemäß wird dies durch die Gegenstände in den Patentansprüchen erreicht.

### Summarische Beschreibung / Lösung

Die vorliegende Erfindung beruht auf den Erkenntnissen der Anmelder, dass durch gleichzeitigen Nachweis von mindestens zwei molekularen Markern, nämlich krankheits-assoziierten Veränderungen von Genexpressionen bzw. viralen Nukleinsäuren, die Spezifität des Nachweises von krankhaft veränderten Zellen in Gebärmutterhalsabstrichen, z.B. Karzinomzellen und deren Vorstufen, erhöht wird und auf Grund der Aussagekraft kombinierter Markerfärbungen eine spezifischere und darüber hinaus auch eine automatisierbare Detektion ermöglicht.

### Detaillierte Beschreibung:

Die Erfindung bezieht sich auf das Verfahren gemäß Anspruch 1, welches molekulare Markernutzt, die bei Einzelnachweisen eine nicht ausreichende Spezifität hinsichtlich der Erkennung von krankhaft veränderten Zellen oder Geweben erreichen, da sie zum Teil auch in nicht krankhaft verändertem biologischen Material in ähnlicher oder unterschiedlicher Menge vorhanden sind. Dies basiert auf der Tatsache, dass diese Marker Proteine sein können, die an physiologischen Regulationsprozessen auch in gesunden Zellen beteiligt sind. Darüber hinaus kann der Nachweis des molekularen Markers aufgrund unspezifischer Kreuzreaktion des Antikörpers nicht eindeutig sein und äußert sich in der Anfärbung von Partikeln des biologischem Materials, die den molekularen Marker nicht beinhalten. Die Erfinder haben die Beobachtung gemacht, dass durch den gleichzeitigen Nachweis von mindestens zwei Markern in einer Zelle, die mangelnde Spezifität des Einzelmarkernachweises kompensiert werden kann, so dass eine höhere Spezifität beim Nachweis von abnormalen Zellen bzw. Gewebeabschnitten gewährleistet ist. Somit ist durch die Kombination mehrerer Marker in einer Zelle bei der Diagnostik von biologischen Proben die Aussagekraft größer als bei der Verwendung von Einzelmarkern.

Die Markerkombinationen beinhalten die Visualisierung von Genexpressionen mindestens einer der nachfolgend aufgeführten Genklassen: Onkogene, Tumorsuppressorgene, Apoptosegene, Proliferationsgene, Reparaturgene und Virusgene, bzw. die einer veränderten Genexpression aus mindestens einer der aufgeführten Genklassen in Kombination mit der Visualisierung viraler Nukleinsäuren. Bevorzugte Kombinationen enthalten folgende molekulare Marker: Her2/neu, p16, p53, MN, mdm-2, bcl-2 und EGF-Rezeptor und DNA der HPV Subtypen 6, 11, 16, 18, 30, 31, 33, 35, 45, 51, 52 . Besonders bevorzugt sind folgende Kombinationen: her2/neu mit p16 oder EGF-R mit p16 oder p53 mit her2/neu oder her2/neu mit mdm-2 oder bcl-2 mit p16 oder bcl-2 mit her2/neu oder p16 mit p53.

Erfindungsgemäß werden die Erkenntnisse der Anmelder für ein Verfahren zur frühen Diagnose krankheitsassoziierter Zellen bzw. Gewebeabschnitte genutzt, das die Detektion von Kombinationen der beschriebenen molekularen Marker mit dem Ziel einer automatisierten Detektion von Karzinomen und ihrer Vorstufen umfasst. Die automatisierte und spezifische Detektion von Tumorzellen kann durch folgendes Verfahren gewährleistet werden: Zum einen müssen mindestens zwei Signale in einer Zelle vorhanden sein, zum anderen muss das Signal der beiden Marker jeweils über einer individuell definierten Intensität bzw. einem individuell definierten Schwellwert liegen. Durch die Anwendung dieser beiden Kriterien kann man jene Zellen als gesund betrachten, welche z.B. einen Marker über der gesetzten Schwelle exprimieren oder eine Färbung beider Marker unterhalb der jeweilig definierten Signalstärke aufweisen.

Der Ausdruck "Molekularer Marker" umfasst molekulare Veränderungen von Zellen, insbesondere Veränderungen der Genexpression, die im Zusammenhang mit einer veränderten oder krankhaften Zellbeschaffenheit beobachtet worden sind. Verfahren zum Nachweis molekularer Marker umfassen jegliche Verfahren, die die Quantität oder Präsenz der Marker bestimmen, entweder auf Nukleinsäure- oder Proteinebene. Für den Nachweis auf Proteinebene bietet sich die Verwendung von Antikörpern oder anderen spezifischen Bindungsproteinen (z.B. Anti-Cullinen) an, die einen anschließenden cytochemischen oder histochemischen Nachweis mit chromogener und/oder fluoreszenter Detektion erlauben. Für den Nachweis auf Nukleinsäureebene bieten sich Hybridisierungstechnologien an, die u.U. auch nach zusätzlichen Verstärkungsschritten (z.B. immuncytochemischer Amplifikation von markierten Sonden nach Bindung an die Zielsequenzen) ebenfalls mit cytochemischen oder histochemischen Färbereaktionen nachgewiesen werden können.

Der Ausdruck "gleichzeitiger Nachweis von mindestens zwei molekularen Markern" umfasst Verfahren, die die Expression von mindestens zwei Genen in einer Körperprobe, insbesondere auch in einer einzelnen Präparation der Körperprobe, bevorzugt in einer einzelnen Zelle, erkennbar machen, so dass die Genexpressionen in Zusammenhang miteinander betrachtet werden können.

Der Ausdruck "Aussagekraft kombinierter Markerfärbungen" umfasst die Verknüpfung von mindestens zwei Informationsinhalten, die auf Grund des Nachweises von mindestens zwei Markern in einer Körperprobe, bevorzugt in einer einzelnen Zelle, gewonnen wurden. Zusätzlich können durch Definition von Schwellwerten der Markerintensitäten gesunde Zellen spezifisch von krankhaften Zellen unterschieden werden.

Der Ausdruck "krankhaft veränderte Zellen von Gebärmutterhalsabstrichen" umfasst Karzinome und ihre Vorstufen aus dem Utero-Vaginal-Trakt der Frau, welche durch eine gynäkologische Routineentnahme auf einen Objektträger aufgebracht werden oder im Durchfluß-Fluoreszenzmeßgerät (FACS) vermessen werden.

Der Ausdruck "automatisierbare Detektion" umfasst Verfahren, die die manuelle Arbeitskraft von menschlichem Personal ganz oder auch nur in Teilschritten ersetzt und insbesondere bei Schritten des Nachweisverfahrens oder bei der anschließenden Dokumentation oder Informationsverarbeitung Verwendung finden. Dies beinhaltet Schritte der Probenaufbereitung, Probenfärbung, Probenauslesung und Informationsverarbeitung. Die Detektion kann dabei sowohl über Absorptions-, Reflexions- oder Fluoreszenzmessungen erfolgen.

Der Ausdruck "diagnostisches Expertensystem" umfaßt eine Computersoftware, die die Bildinformationen in einen Diagnosevorschlag umwandelt. Dieses Expertensystem kann die gesamte vorhandene Information oder Teile davon aufgrund von in der Software vorhandenen Parametern oder von externen Informationen, auf die die Software zugreifen kann, zu einem Diagnosevorschlag konsolidieren. Sollten zur Absicherung des Diagnosevorschlags weitere Parameter erforderlich sein, kann die Software die Erhebung dieser Parameter vorschlagen oder durch Kopplung mit geeignten Analysegeräten im Sinne eines Reflex-Algorithmus selbsttätig anfordern.

Der Ausdruck "Verstärkungssystem" umfaßt biochemische Verfahren, die die Signalintensitäten erhöhen, so daß ein für den spezifischen Nachweis eines molekularen Markers günstigeres Signal/Rausch-Verhältnis erzeugt wird. Dies wird üblicherweise durch Einsatz von zusätzlichen Antikörpern und/oder enzymatischer Nachweisreaktionen erreicht.

Mit der vorliegenden Erfindung ist es möglich, krankhaft veränderte Zellen, z.B. Karzinome und ihre Vorstufen des Gebärmutterhalses spezifisch zu detektieren. Ebenfalls offenbart ist ein Kit zur Durchführung eines erfindungsgemäßen Verfahrens, das folgende Bestandteile enthält:
(a) Reagenzien zum Nachweis von mindestens zwei der molekularen Marker, nämlich markierte und/oder unmarkierte Antikörper gegen Her2/neu, p16, p53, MN, mdm-2, bcl-2, EGF-Rezeptor und HPV L1, sowie markierte DNA-Sonden die Bereiche des viralen HPV-Genoms enthalten.
(b) Übliche Hilfsmittel, wie Puffer, Träger, Signalamplifikationssubstanzen, Färbereagenzien, etc.
(c) Automatisierte Verfahren der Probenaufbereitung, -färbung und Signaldetektion
(d) Protokolle und Reagenzien zur Anfärbung von Zelllinien als Kontrollreaktion Für die einzelnen Komponenten des Kits gelten die vorstehenden Ausführungen. Einzelne oder mehrere Komponenten des Kits können auch in veränderter Form Verwendung finden.

Mit der vorliegenden Erfindung ist es möglich Karzinome und ihre Vorstufen in Gebärmutterhalsabstrichen manuell, aber auch in teil- oder vollautomatisierten Verfahren zu detektieren. Da die erfindungsgemäß erzielten Ergebnisse des gleichzeitigen Nachweises von mindestens zwei molekularen Markern nicht einer subjektiven Bewertung unterliegen, sondern vielmehr einer objektiven, automatisierten Detektion von krankhaften Veränderungen in biologischen Materialien förderlich sind, können die morphologischen Befunde, beispielsweise die eines Pap-Tests, durch objektive Parameter, z.B. auch in Form von "Reflex-Testing", ergänzt werden. Aufgrund der schnellen und automatisierten Handhabbarkeit der Verfahren sind sie für große, kosten- und personalsparende Screening-Verfahren geeignet.

Somit stellt die vorliegende Erfindung einen wichtigen Beitrag zur spezifischen Detektion von Tumorzellen und deren Vorläufern bei der Früherkennung des Gebärmutterhalskrebses in der Diagnostik von Abstrichen dar.

### Beispiele

Nachfolgend werden beispielhaft Protokolle zur Durchführung der beschriebenen Erfindung gegeben. In diesen Beispielen werden exakte Reaktionsbedingungen für die jeweiligen Antikörper bzw. DNA-Sonden angegeben, allerdings können verschiedene Parameter, wie z.B. Inkubations- und Waschtemperatur, Inkubationsund Waschzeiten sowie die Konzentration von Antikörpern und anderen Reagenzien, in Abhängigkeit von den jeweiligen Antikörpern bzw. DNA Sonden variiert werden. Ebenso können hier beschriebene Verstärkungssysteme weggelassen werden oder neu hinzugefügt werden.

### Versuchsbeschreibung zum simultanen Nachweis von zwei molekularen Markern mit spezifischen Antikörpern auf Cervix-Abstrichen

Durch Präparationstechniken wie Thin-Prep (Firma Cytyc) werden die Zellen, die in Preservcyt (Firma Cytyc) gelagert werden, auf Objektträger (OT) aufgebracht. Mit kaltem Ethanol werden die Zellen 3 Minuten fixiert, danach wäscht man die Objektträger in PBS (137 mM NaCl, 3 mM KCl, 4 mM Na₂HPO₄, 2 mM, KH₂PO₄). Nach Blockierung unspezifischer Bindungsstellen mit 5 % fötalem Kälberserum in PBS-Puffer für 30 min bei Raumtemperatur (RT) werden die OT für 60 min in einer feuchten Kammer gleichzeitig mit zwei spezifischen, monoklonalen Antikörpern (her2/neu (Klon 3B5, 20 µg/ml) und p16 (Klon DCS 50.1, 10 µg/ml) von Oncogene Science/BAYER) bei RT inkubiert. Einer der Antikörper ist mit Fluorescein, der andere mit Biotin gekoppelt. Nach jedem der folgenden Inkubationsschritte werden die OT jeweils 3 mal 5 min mit PBS gewaschen. Zum Nachweis der einzelnen Antigen-Antikörper-Bindungen werden nacheinander zwei unterschiedliche Färbesysteme angwendet. Als erstes Färbesystem inkubiert man 30 min mit Streptavidin-Cy3 (Mobi Tec, 5 µg/ml in PBS mit 5% Kälberserum), danach mit anti-Streptavidin-Biotin (Vector; 2 µg/ml in PBS mit 5% Kälberserum) für weitere 30 min, um eine Verstärkung des Signals zu erreichen. Zuletzt inkubiert man noch mal mit Streptavidin-Cy3. Das zweite Färbesystem besteht aus dem Alexa Fluor 488 Signal-Amplification Kit™ (Molecular Probes, Bestell-Nr. A-11053 ). Zuletzt werden die OT mit Mowiol™ (Hoechst) eingedeckelt.

### Versuchsbeschreibung zum simultanen Nachweis von drei molekularen Markern mit spezifischen Antikörpern auf Cervix-Abstrichen

Durch Präparationstechniken wie Thin-Prep (Firma Cytyc) oder Cyto-Spin (Firma Shandon) werden die Zellen, die z.B. in Preservcyt (Firma Cytyc) gelagert werden, auf Objektträger (OT) aufgebracht. Mit kaltem Ethanol werden die Zellen 3 Minuten fixiert, danach wäscht man die Objektträger in PBS (137 mM NaCl, 3mM KCl, 4mM Na₂HPO₄, 2 mM, KH₂PO₄, pH 7,4). Nach Blockierung unspezifischer Bindungsstellen mit 5 % fötalem Kälberserum mit 4 mmol/l Levamisole (Firma Sigma) in PBS-Puffer für 30 min bei Raumtemperatur (RT) werden die OT für 60 min in einer feuchten Kammer gleichzeitig mit drei spezifischen, monoklonalen Antikörpern (her2/neu (Klon 3B5, 20 µg/ml), bcl-2 (Klon 100, 2 µg/ml) und p16 (Klon DCS 50.1, 10 µg/ml) von Oncogene Science/BAYER) bei Raumtemperatur inkubiert. Der erste Antikörper ist mit Fluorescein, der zweite mit Digoxigenin und der dritte mit Biotin gekoppelt. Nach jedem der folgenden Inkubationsschritte werden die OT jeweils 3 mal 5 min mit PBS gewaschen. Zum Nachweis der einzelnen Antigen-Antikörper-Bindungen werden nacheinander drei unterschiedliche Färbesysteme angwendet. Als erstes Färbesystem inkubiert man 30 min mit Streptavidin-Cy3 (Mobi Tec, 5 µg/ml in PBS mit 5% Kälberserum mit 4 mmol/l Levamisole), danach mit biotinyliertem anti-Streptavidin Antikörper (Vector; 2 µg/ml in PBS mit 5% Kälberserum mit 4 mmol/l Levamisole) für weitere 30 min, um eine Verstärkung des Signals zu erreichen. Zuletzt inkubiert man noch mal mit Strepavidin-Cy3. Das zweite Färbesystem besteht aus dem Alexa Fluor 488 Signal-Amplification Kit™ (Molecular Probes, Bestell-Nr. A-11053). Für das dritte Färbesystem inkubiert man mit einem Konjugat aus anti-Digoxigenin Antikörper vom Schaf und Alkalischer Phosphatase (DAKO, 1:150 verdünnt in PBS mit 5% Kälberserum mit 4 mmol/l Levamisole) für 30 min. Die fluoreszente Auslesung wird mit dem ELF97 Cytological Labeling Kit (Bestell-Nr. E 6602) von Molecular Probes nach Angaben des Herstellers durchgeführt. Zuletzt werden die OT mit Mowiol™ (Hoechst) eingedeckelt.

### Versuchsbeschreibung zum simultanen Nachweis von zwei molekularen Markern auf Cervix-Abstrichen mit einem spezifischen Antikörper und einer HPV DNA Sonde

Bei der Kombination von einer immunocytochemischen Antikörperfärbung mit einer DNA *in situ* Hybridisierung wird die Probenpräparation, -fixierung und Blockierung sowie die Inkubation mit einem Primärantikörper wie im oben beschriebenen Protokoll für Antikörperfärbung durchgeführt. Der primäre Antikörper ist ein Fluorescein gekoppelter Antikörper gegen das Protein p16 (Klon DCS 50.1) von Oncogene Science/BAYER und wird in einer Konzentration von 10 µg/ml eingesetzt. Zum Nachweis der Antigen-Antikörper-Bindung wird zunächst mit einem Sekundärantikörper, Kaninchen anti-FITC (MoBiTec) in einer Konzentration von 15µg/ml, und anschließend mit einem Ziege anti-Kaninchen Antikörper gekoppelt an Alkalische Phosphatase von Dianova in einer Verdünnung 1:100 in PBS, für 30 min. bei Raumtemperatur (RT) inkubiert. Als Färbesystem wird 5 min bei Raumtemperatur mit dem chromogenen alkalische Phosphatase Substrat Fuchsin von DAKO nach Angaben des Herstellers inkubiert. Zur Fixierung der Färbung wird 5 min. mit 4% Paraformaldehyd in PBS nachfixiert. Daran schließt sich die *in situ* Färbung an. Dazu werden die OT zunächst 2 mal in 2xSSC Puffer (0,3M NaCl, 30mM Na-citrat) gewaschen und anschließend für 60 min bei 37°C mit 40µg/ml RNase in 2xSSC verdaut. Nach 2 zweiminütigen Waschschritten mit PBS wird für 10 min mit 2µg/ml Proteinase K bei 37°C verdaut. Nach 3 x 2 min Waschen mit destilliertem Wasser, werden 20µl gebrauchsfertige Digoxigenin markierte HPV16 DNA-Sonde (Kreatech) in Hybridisierungspuffer (Kreatech) auf die OT gegeben, mit Deckgläschen abgedeckt, abgedichtet, für 8 min auf 94°C zur Denaturierung erhitzt und anschließend über Nacht bei 37°C inkubiert. Nach Abnahme der Hybridisierungslösung wird erneut mit 3% Kaninchen Serum in Boehringer Blocking Puffer (0,05g Blocker (Boehringer 1201107) in 5ml Blockpuffer (150 mM NaCl, 100 mM Tris-HCl, pH 7,5)) für 30 min blockiert. Für die chromogene Auslesung werden nacheinander mit DAKO Kaninchen anti-Digoxigenin Antikörper gekoppelt mit Peroxidase (1:150 verdünnt mit Blockpuffer), Dig-Tyramid (5,71µg/ml) (hergestellt wie beschrieben in: Hopman et al., 1998, J Histochem Cytochem., 46(6), 771-777) in PBS/0,1M Imidazol pH 7,6/0,001% H₂O₂ und wiederum mit Kaninchen anti-Digoxigenin Antikörper gekoppelt mit Peroxidase (DAKO, 1:150 verdünnt in TBST (50mM Tris/HCl, 0,3M NaCl, 1% Tween 20, pH 7,6) mit 3% Kaninchenserum) für jeweils 15 min bei RT inkubiert. Dazwischen wird jeweils 3 x 5 min mit PBST gewaschen. Für die chromogene Farbreaktion wird mit DAB Substrat von DAKO nach Angaben des Herstellers für 10 min gefärbt. Zuletzt werden die OT mit Mowiol™ eingedeckelt.

### Versuchsbeschreibung zur automatisierten Detektion abnormaler Zellen, die durch simultanen Nachweis von mindestens zwei molekularen Markern angefärbt wurden

Cervix-Abstriche, bei denen mindestens zwei molekulare Marker nach den oben beschriebenen Methoden mit Antikörpern nachgewiesen wurden, werden mittels eines Fluoreszenzmikroskops mit einem ansteuerbaren Kreuztisch für bis zu 8 Objektträger (Olympus AX70 mit Multicontrol-Box 2000-3 und analySIS Treibersoftware "Modul Stage^{m}"), einer modifizierten Version der analySIS 3.0 Software der Firma Soft Imaging Systems GmbH (SIS), die als SIS-Paket "Grabbit Dual Pro" zusätzliche Module, insbesondere ein FFT- (="Fast Fourier Transformation"), ein MIA- (="Multiple Image Alignment") und ein C-Modul enthält, ausgewertet. Für die Bildaufnahmen werden hochauflösende Schwarz-Weiß-(MV2 Slow Scan Kamera, 12 Bit von SIS) und Farbkameras (DXC-950P 3CCD Chip von Sony) verwendet. In Kombination sind diese Systeme für eine 16 Bit Grautonbildanalyse und zur Farbbildanalyse im RGB- und HSI-Farbraum bis 24 Bit Bildtiefe geeignet.

Die Position von insgesamt bis zu acht Objektträgern wird nach Kalibrierung des ansteuerbaren Kreuztisches (analySIS "Modul Stage") durch Definition von acht aufeinanderfolgenden Tischwegen im Menü "Automation" (analySIS "Modul Grains") relativ zum "logischen Nullpunkt" erfaßt. Die Gesamtfläche der biologischen Proben, die sich auf Grund der automatisierten Thin-Prep (Cytyc) Methodik immer in einem definierten Bereich der Objektträger befinden, wird flächendeckend in Einzelbereiche einer Größe von 571,2µm x 457,96µm zerlegt, die dem Bildausschnitt eines Photos bei 20facher Vergrößerung mit der mikroskopischen Einheit entsprechen. Jede biologische Probe wird durch einen Tischweg analysiert. Jeder Tischweg besteht aus 750 einzelnen Tischwegpositionen (30 Zeilen, 25 Spalten), die das Mikroskop meanderförmig horizontal als Serie von Tischwegpositionen abfährt, so dass jeder Bereich der biologischen Probe der jeweiligen Objektträgerposition durch eine Tischwegposition "Stoß an Stoß" und nicht überlappend erfaßt ist. Für jedes Präparat auf dem Arbeitstisch ist somit jeweils ein identischer Tischweg definiert. Die Addition der 8 Tischwege im Menü "Automation" ermöglicht die automatisierte Analyse aller auf dem Arbeitstisch positionierten Präparate.

Generell wird jede Tischwegposition nach Anregung mit den 3 verschiedenen Fluoreszenzwellenlängen jeweils 1 x mit der hochauflösenden S/W-Kamera und entsprechenden Fluoreszenzfarbfiltern photografiert. Die Fluoreszenzintensitäten der Einzelphotos werden daraufhin vermessen. Dies geschieht mittels der Registrierkarte "Messung" im Menü "Automation". Durch Übereinanderlagerung von blauen, roten und grünen Einzelbildern werden Farbmischungen erzeugt, die mit den Fluoreszenzintensitäten der Einzelbilder an identischen Tischwegpositionen korrelieren. Es erfolgt die "Phasenanalyse", das heißt die Bereiche bestimmter Farbmischungen werden vermessen und als Fläche quantitativ wiedergegeben. Bestimmte Farbmischungen sind dabei für krankhaft veränderte Zellen charakteristisch, so daß eine diagnostische Aussage getroffen werden kann.

Im Einzelnen wird für die Fluoreszenzmessungen mit mehreren molekularen Markern pro Tischwegposition für jeden Fluoreszenzfilterkanal ein Schwarz-Weiss-Photo gemacht. Den Einzelbildern einer Tischwegposition werden daraufhin Farben zugeordnet und die so entstehenden Falschfarbenbilder werden mittels des "FIP-Moduls" übereinandergelegt, so daß sich durch "Addition" der unterschiedlichen Fluoreszenzfarben Farbmischungen in Abhängigkeit zu den Fluoreszenzintensitäten ergeben. Beispielsweise kommt es bei gleichzeitiger und gleichstarker Fluoreszenzfärbung im roten und grünen Farbkanal zur Bildung einer gelben Mischfarbe an der entsprechenden Bildposition. Je nach Stärke der Fluoreszenzintensitäten können die Mischfarben auch ins Rot bzw. Grün tendieren. In ähnlicher Weise kommt es bei der Überlagerung von blauen, grünen und roten Fluoreszenzen gleicher Intensität zur Bildung einer weißen Mischfarbe. Diese überlagerten Bilder werden daraufhin mittels Schwellwertsetzung und anschließender "Phasenanalyse" vermessen, wobei dies direkt durch Farbschwellwertsetzung im RGB-Modus (Menü Bilder) erfolgt. Diese Schritte lassen sich manuell ausführen, können aber auch automatisiert durchgeführt werden, indem Makros im Menü "Extras" unter "Makros aufzeichnen" erstellt und anschließend im Menü "Automation" abgerufen werden. Letztendlich erhält man für bestimmte Farbmischungen eine quantitative Aussage in Form einer Flächenangabe (= Fläche auf der biologischen Probe, die auf Grund der Anfärbung mit mindestens 2 molekularen Markern mit bestimmter Intensität eine genau definierte Mischfarbe aufweist). Mit der Registrierkarte "Protokoll" im Menü "Automation" können sowohl die analysierten Bilder als auch die Flächenangaben für die jeweiligen Tischwegpositionen automatisch abgespeichert werden. So erhält man für jede Tischwegposition eine quantitative Aussage über die in diesem Bereich gemessenen Fluoreszenzintensitäten bzw. Farbmischungen. Die Werte der Farbmischungen der einzelnen Tischwegpositionen (= Übereinanderlagerung von drei Einzelphotos) werden für jeden Tischweg (= Einzelpräparat) in jeweils einer Excel-Datei wiedergegeben. Die Exceltabellen bestehen demzufolge aus 750 Zeilen für die 750 einzelnen Tischwegpositionen pro Tischweg und aus 8 Spalten für die 8 unterschiedlichen Farbmischungen. Da bestimmte Farbmischungen (z.B. gelbe Farbmischungen bei der Darstellung von zwei Einzelmarkern in Rot und Grün) auf die gleichzeitig hohe Expression mehrerer krankheitsassoziierter Marker zurückzuführen sind, werden diese Tischwegpositionen als "krankhaft" markiert (= "Flagging"), so daß die entsprechenden Fluoreszenzaufnahmen später als Einzelbilder zur Kontrolle visuell begutachtet werden können. Die in einer Exceltabelle für die acht Farbmischungen angegebenen Flächen werden mittels eines Makros in Excel verrechnet, d.h. sämtliche Werte einer Spalte (= eine bestimmte Farbmischung) werden aufsummiert. Somit erhält man für ein biologisches Präparat (= Tischweg) einen Zahlenwert für jede der acht Mischfarben. Überschreitet die Summe von bestimmten Farbmischungen, die für krankhaft veränderte Zellen charakteristisch sind (wie z.B. die oben bereits erwähnte gelbe Farbmischung), einen kritischen Mindestwert, wird die durch den entsprechenden Tischweg analysierte biologische Probe bzw. der jeweilige Cervix-Abstrich als krankhaft eingestuft. Somit gelangt man über die quantitative Analyse der verrechneten Einzelfluoreszenzintensitäten automatisch zu einer diagnostischen Aussage für die biologische Probe.

### Versuchsbeschreibung zum simultanen Nachweis von zwei molekularen Markern mit spezifischen Antikörpern im Durchflußzytometer

Abstrichzellen, die in Preservcyt (Cytyc) gelagert sind, werden zunächst durch ein Nylontuch mit 100 µm großen Poren gepreßt, um Zellklumpen zu vereinzeln. Nach Sedimentierung durch Zentrifugation wird 1 x mit PBS gewaschen und anschließend mit einer der nachfolgenden Methoden permeabilisiert: a) OPF Methode (ORTHOPermeaFixTM b) F&P (FIX&PERM Cell Permeabilization Kit, Imtec) c) MWH (microwave heating). Alle Methoden sind entsprechend der Herstellerangaben bzw. nach Millard et al (Clin Chem 1998, 44, 2320-2330) durchzuführen. Die Inkubation der permeabilisierten Zellen mit den beiden Maus-Antikörpern (her2/neu (Klon 3B5, 20 µg/ml), und p16 (Klon DCS 50.1, 10 µg/ml) von Oncogene Science/BAYER) erfolgt für 60 min. Der erste Antikörper ist mit Fluorescein, der andere mit Biotin gekoppelt. Nach jedem der folgenden Inkubationsschritte werden die Zellen sedimentiert und insgesamt 2 mal mit PBS gewaschen. Zum Nachweis der einzelnen Antigen-Antikörper-Bindungen werden nacheinander zwei unterschiedliche Färbesysteme angwendet. Für das erste Färbesystem inkubiert man 30 min mit Streptavidin-Cy3 (Mobi Tec, 5 µg/ml). Das zweite Färbesystem besteht aus dem Alexa Fluor 488 Signal-Amplification Kit™ (Molecular Probes, Bestell-Nr. A-11053). Anschließend werden die Zellen in ISOTON II (Coulter) aufgenommen und in einem Durchflußzytometer (FACScan, Becton Dickinson) vermessen, welches mit Laser-Anregung für die grüne und rote Fluoreszenz ausgestattet ist. Die Fluoreszenzintensitäten können graphisch dargestellt werden. Die relative und absolute Anzahl der negativen, der einfach positiven bzw. der doppelt positiven Zellen kann nach Definition von Schwellwerten der Signalintensitäten dargestellt werden. Ein diagnostisches Expertensystem wandelt diese Information in einen Diagnosevorschlag um.

## Patentansprüche

1. Verfahren zur Detektion von Tumorzellen und deren Vorläufern in Gebärmutterhalsabstrichen durch simultane Detektion von mindestens zwei molekularen Markern aus Farbmischungen von markierten Reagenzien in einer Zelle, wobei mindestens zwei Signale in einer Zelle vorhanden sein müssen, wobei zum anderen des Signal der beiden Marker jeweils über einer individuell definierten Intensität bzw. einem individuell definierten Schwellwert liegen muss, wobei die Reagenzien Antikörper oder Nukleinsäuren sind, und die Antikörper oder Nukleinsäuresonden direkt oder indirekt durch unterschiedlich fluoreszierende oder chromogene Farbsubstanzen ausgelesen werden,
wobei mindestens einer der nachfolgenden Marker in der Kombination enthalten ist:her2/neu, p16, p53, mdm-2, bcl-2, EGF-Rezeptor, spezifische DNA der HPV Subtypen 6, 11, 16, 18, 30, 31, 33, 35, 45, 51, 52,
**dadurch gekennzeichnet, dass** nach Anregung bei den verschiedenen Fluoreszenzwellenlängen jeweils 1x mit einer S/W-Kamera und entsprechenden Fluoreszenzfarbfiltern fotografiert wird, durch Übereinanderlagerung der Einzelbilder Farbmischungen erzeugt werden, die mit den Fluoreszenzintensitäten der Einzelbilder korrelieren, wobei für die Farbmischungen eine quantitative Aussage erhalten wird in Form einer Flächenangabe, die der Fläche auf einer Gebärmutterhalsabstrichprobe entspricht, die aufgrund der Anfärbung mit den mindestens zwei molekularen Markern mit bestimmter Intensität die Mischfarbe aufweist und wobei die Farbmischung für krankhaft veränderte Zellen charakteristisch ist, so dass eine diagnostische Aussage getroffen werden kann.

2. Verfahren gemäß Anspruch 1 zur Detektion von Tumorzellen und deren Vorläufern in Gebärmutterhalsabstrichen **gekennzeichnet durch** simultane Detektion von drei molekularen Markern aus Farbmischungen in einer Zelle.

3. verfahren nach Anspruch 2, **dadurch gekennzeichnet, dass** die Markerkombination her2/neu und p16 und bcl-2 ist und die Reagenzien Antikörper sind.

4. Verfahren nach einem der Ansprüche 1 - 3, welches eine automatisierbare Detektion ermöglicht.

5. Verfahren nach Anspruch 4, **dadurch gekennzeichnet, dass** die automatische Informationsverarbeitung mit einem diagnostischen Expertensystem verbunden ist, das die Bildinformation zu einem Diagnosevorschlag konsolidiert und gegebenenfalls ein Reflex-Testing ermöglicht.

## Claims

1. Method for detecting tumour cells and their precursors in uterine cervical smears by simultaneous detection of at least two molecular markers from colour mixtures of marked reagents in a cell, wherein at least two signals must be present in a cell, wherein on the other hand the signal of each of the two markers must exceed an individually defined intensity or an individually defined threshold value,
wherein the reagents are antibodies or nucleic acids, and the antibodies or nucleic acid probes are read out directly or indirectly by means of differently fluorescent or chromogenic dye substances,
wherein at least one of the following markers is included in the combination: her2/neu, p16, p53, mdm-2, bcl-2, EGF receptor, and specific DNA of the HPV subtypes 6, 11, 16, 18, 30, 31, 33, 35, 45, 51 and 52,
**characterised in that,** following excitation at the different fluorescence wavelengths, a photograph is taken once in each case using a B/W camera and appropriate fluorescence colour filters, colour mixtures which correlate with the fluorescence intensities of the individual images are created by overlaying the individual images, wherein a quantitative assessment for the colour mixtures is obtained in the form of an area value which corresponds to the area on a cervical smear sample which, due to having been stained with the at least two molecular markers at a specific intensity, exhibits the mixed colour and wherein the colour mixture is characteristic of pathologically altered cells, thereby enabling a diagnostic prognosis to be made.

2. Method according to claim 1 for detecting tumour cells and their precursors in uterine cervical smears, **characterised by** simultaneous detection of three molecular markers from colour mixtures in a cell.

3. Method according to claim 2, **characterised in that** the marker combination is her2/neu and p16 and bcl-2 and the reagents are antibodies.

4. Method according to one of claims 1 - 3, which enables detection to be performed in an automated manner.

5. Method according to claim 4, **characterised in that** the automatic information processing is connected to a diagnostic expert system which enables the image information to be consolidated into a proposed diagnosis and if necessary enables reflex testing.

## Revendications

1. Procédé de détection de cellules tumorales et de leurs précurseurs dans des frottis du col de l'utérus par détection simultanée d'au moins deux marqueurs moléculaires à partir de mélanges colorés de réactifs marqués dans une cellule, au moins deux signaux devant être présents dans une cellule, dans lequel pour l'autre du signal des deux marqueurs il doit se trouver respectivement au-dessus d'une intensité définie individuellement ou d'une valeur de seuil définie individuellement, les réactifs étant des anticorps ou des nucléiques et les anticorps ou les sondes d'acide nucléique étant lues directement ou indirectement par des substances colorées fluorescentes chromogènes ou différentes,
dans lequel au moins l'un des marqueurs suivants contenu dans la combinaison : her2/nouveau, p16, p53, mdm-2, bcl-2, récepteur EGF, ADN spécifique des sous-types 6, 11, 16, 18, 30, 31, 33, 35, 45, 51, 52 d'HPV,
**caractérisé en ce qu'**après excitation aux longueurs d'onde de fluorescences différentes respectivement de 1x, on photographie par un appareil photographique S/W et avec des filtres colorés de fluorescence adéquats, on produit par superposition des images individuelles des mélanges colorés qui sont corrélés aux intensités de fluorescence des images individuelles en obtenant, pour les mélanges colorés, une prédiction quantitative sous la forme d'une indication de surface, qui correspond à la surface d'un échantillon de frottis du col de l'utérus qui, sur la base de la coloration par les au moins deux marqueurs moléculaires d'une intensité déterminée, a la couleur mélangée et dans lequel le mélange de couleur est caractéristique de cellules modifiées par la maladie de sorte que l'on peut donner un diagnostic.

2. Procédé suivant la revendication 1 de détection de cellules tumorales et de leurs précurseurs dans des frottis du col de l'utérus, **caractérisé par** une détection simultanée de trois marqueurs moléculaires à partir de mélanges colorés dans une cellule.

3. Procédé suivant la revendication 2 de détection de cellules tumorales et de leurs précurseurs dans des frottis du col de l'utérus, **caractérisé en que** la combinaison de marqueurs est her2/nouveau et p16 et bcl-2 et les réactifs sont des anticorps.

4. Procédé suivant l'une des revendications 1 à 3 de détection de cellules tumorales et de leurs précurseurs dans des frottis du col de l'utérus, qui rend possible une détection pouvant être automatisée.

5. Procédé suivant la revendication 4 de détection de cellules tumorales et de leurs précurseurs dans des frottis du col de l'utérus, **caractérisé en ce que** le traitement informatique automatique est relié à un système de diagnostic expert, qui consolide la formation de l'image en un projet de diagnostic et rend possible, le cas échéant, un test réflexe.
